(19) Europäisches Patentamt
European Patent Office
Office européen des brevets

(11) EP 1 191 472 A1

(12) EUROPEAN PATENT APPLICATION

(43) Date of publication:
27.03.2002 Bulletin 2002/13

(51) Int Cl.7: G06F 19/00, G06F 17/60

(21) Application number: 00203290.2

(22) Date of filing: 22.09.2000

(84) Designated Contracting States:
AT BE CH CY DE DK ES FI FR GB GR IE IT LI LU MC NL PT SE
Designated Extension States:
AL LT LV MK RO SI

(71) Applicant: Activa Care AB
223 70 Lund (SE)

(72) Inventor: Nordin, Birgitta
270 52 Borrby (SE)

(74) Representative: Petri, Stellan et al
Ström & Gulliksson AB
Box 41 88
203 13 Malmö (SE)

# (54) Method and system for efficient distribution of resources for care and service needs

(57) A method of measuring need of care and service in elder care and care of the disabled for efficient distribution of resources for need of care and service in for example elder care and care of the disabled, wherein available resources as full-year workers and current needs of each person receiving care are registered, an 0 index for the registered needs is determined for each person receiving care and an evaluation is performed of the level of dependence according to a predetermined scale, a PA index is determined, which relates to a cost counted in the number of full-year workers for the indirect care and transport time, a B index is determined which relates to the work load/full-year worker in relation to applied resources, an average work load is calculated in an N value, which is the total need in relation to available resources, a recommended care resource/person receiving care is calculated, and a recommended care resource/person receiving care is applied.

FIG 3

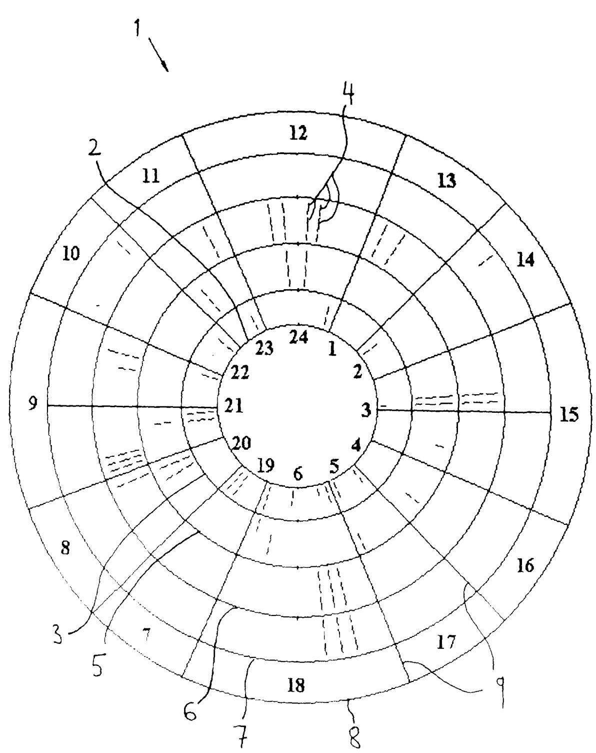

EP 1 191 472 A1

## Description

### Technical Field

The present invention relates to a method and system for optimum distribution of resources and efficient economizing for activities in care and nursing.

### Prior Art

The method for estimating the needs of a person receiving care is based partly on the development steps, a so-called cumulative ordinal scale, from which the functional level of the individual is estimated. By this method a person's ability of managing the daily activities of life is measured and it is determined in which order an individual recovers its ability.

The economical situation in for example town districts under large economy measures and increasing competition results in that it becomes more important to save and distribute available resources equally. One of the most important problems in care and nursing is the difficulty in reflecting the real conditions for personnel and persons receiving care (persons receiving care relates to persons who need help to satisfy physical and social needs according to the care steps and nursing and rehabilitation efforts), in order to apply necessary resources.

This is a problem which creates unbalance and uneven distribution of resources and makes it difficult to survey the activity. It also obstructs the possibility of following up and evaluating the medical and economical development on different levels in the own organization - from the field activity and the individual person receiving care to those who conform to political decisions and the tax payers' commission last. The possibility to follow the health development of the population and perform comparing studies, so-called benchmarking, within and between town districts and county councils in different respects are also limited.

Often, view points have been given from different quarters in town districts and county councils on the distribution of resources in care and nursing and the influence of this for the individual person receiving care. For different reasons the personnel density varies between different parts of the care and nursing activities. The need level of the persons receiving care varies and increases more in some parts. A reason for this in for example Sweden is the purpose of the elder reform, where the possibility to stay at home should exist independent of illness.

Methods for an unbiased reflection of the real conditions for personnel and persons receiving care in care, and elder care and care of the disabled have been missed.

### Summary

It is an object of the present invention to solve the above problem of reflecting the real conditions for personnel and persons receiving care objectively in for example elder care and welfare services for the disabled, and distribute available resources efficiently in order to meet the requirements of care, service and nursing.

This object is achieved by the method according to the characterizing part of claim 1 and the system according to the characterizing part of claim 7.

An advantage of the method and system according to the invention is that the personnel gets fare and more equal conditions, and thereby a better working environment, also resulting in that the individual person receiving care gets an increased and equal quality of care and nursing.

Additionally, the method is adjusted for the individual and follows the development of the individual person receiving care. The method also implies that the development of health for the whole care unit/town district regarding age and sex can be followed and recorded in historical files.

### Short Description of the Drawings

The invention will be described in detail in the following description with reference to the accompanying drawings, in which

FIG 1A shows a first part of the care steps, also called the ADL steps, which relates to physical ADL needs (step 9-4),
FIG 1B shows the other part of the care steps and relates to social needs, so-called instrumental ADL,
FIG 2A shows a form for physical need of care, i.e. steps 9-4 in the care steps,
FIG 2B shows a form for social needs, i.e. steps 3-0 in the care steps,
FIG 2C shows a form for medical needs,
FIG 2D shows a form for rehabilitation needs,
FIG 3 shows a clock of needs,

FIG 4 is a bar graph illustrating care weight index (points) regarding the average of need of care and nursing in home help service in all of the home help groups and blocks of service flats in a town district,

FIG 5 is a bar graph illustrating the care weight (physical and medical needs) in the home help service in all home help groups in a town district for a period of three years,

FIG 6 is a bar graph illustrating (care resource/(person receiving care) and (recommended care resource)/(person receiving care), and

FIG 7 is a bar graph illustrating the work load in points/(full-year worker).

## Description of a preferred embodiment

In the following section an embodiment of a method and system for measuring the need of care, service and nursing in local elder care and welfare service for the disabled, i.e. the total work load, and the follow-up of the need of care, quality and resource utilisation in order to use and distribute available resources for care, service and nursing in an optimum way. The system according to the invention comprises a conventional computer system such as a PC with memory means for storing program instructions and data and a control unit operatively connected to the memory means for the execution of the instructions.

The method according to the invention involves measurement of the ability of an individual to provide for basic physical and secondary social/mental and cultural needs and the ability to self care. The method implies for example that the functional level is estimated according to a scale, based on the biological evolution of the human being, i.e. the development of the child 0-16 (17-) years. By measuring the activities in the development steps, an overview over the functional level of an individual is obtained, i.e. the grade of independence in the performance of the activity. The basic part of the method, to estimate the physical and social needs and the functioning level, i.e. the least common denominator in the care, is still used over the world in a modified/adjusted form.

Further, the needs for medical and rehabilitation efforts are weighted to the above-mentioned physical and social needs. These medical and rehabilitation needs are based on known and well defined indicators.

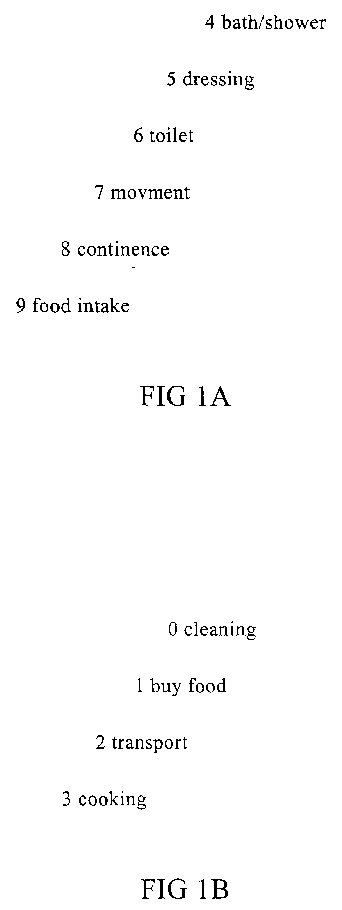

The method according to the invention is based on for example the so-called care steps, wherein the lower and the higher part of the care steps are shown in FIGs 1A and 1B. The primary physical and secondary social needs are registered essentially according to this method. Further, the method implies that a level functioning is estimated according to a scale, which is based on the biological evolution of the human being, (a so-called cumulative ordinal scale), i.e. the development of the child 0-16 years, which is illustrated by the steps 9-4 in FIG 1A and steps 3-0 in FIG 1B.

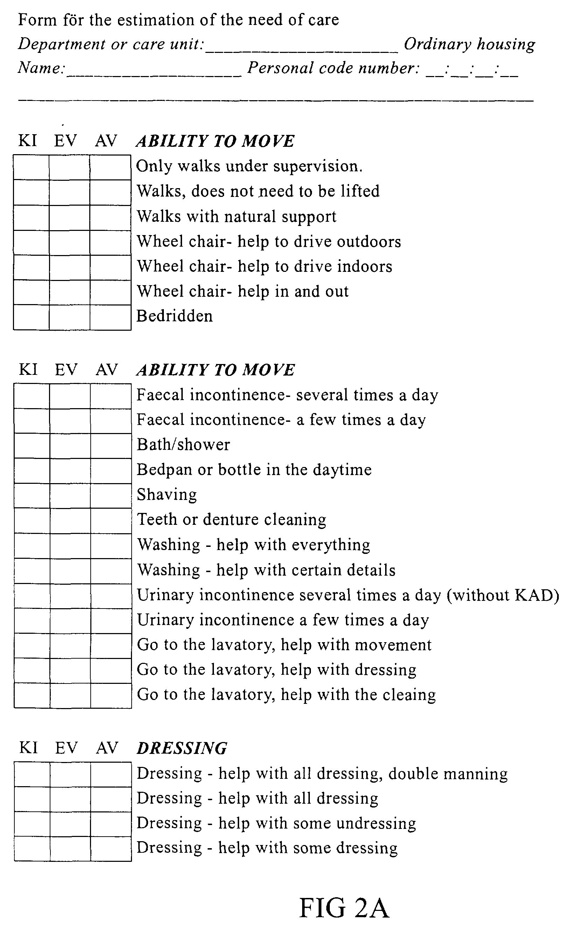

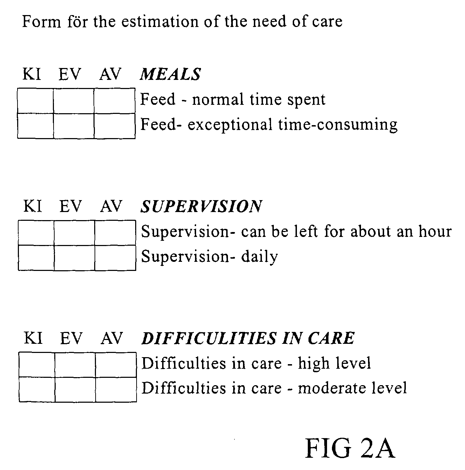

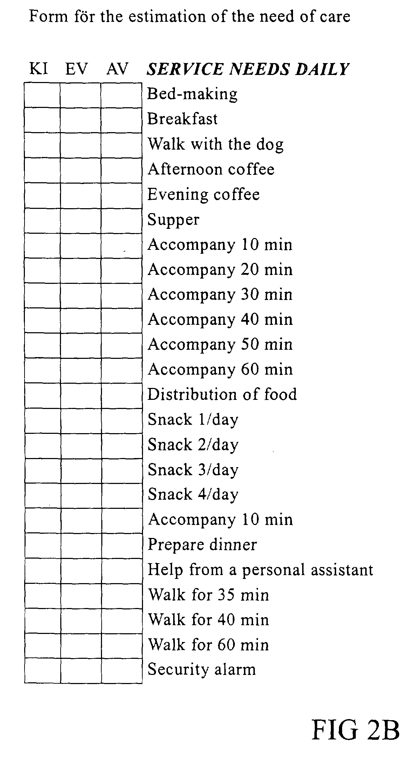

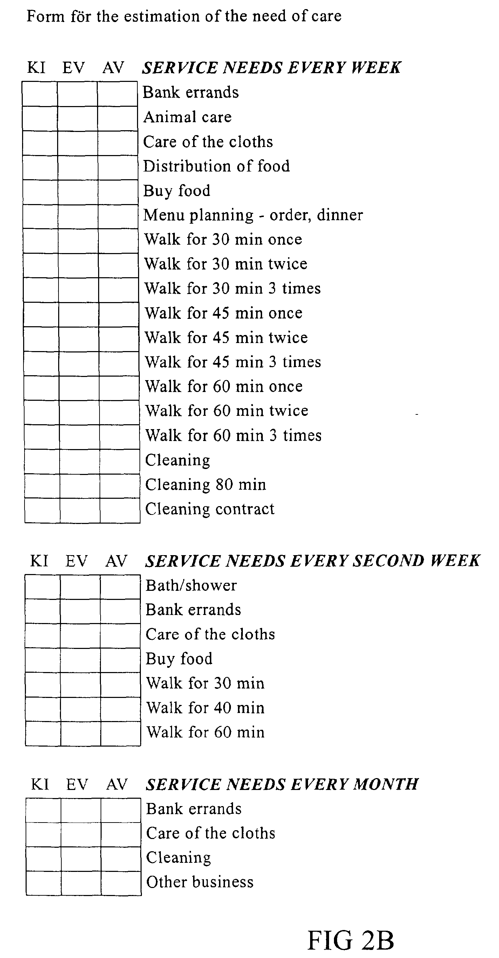

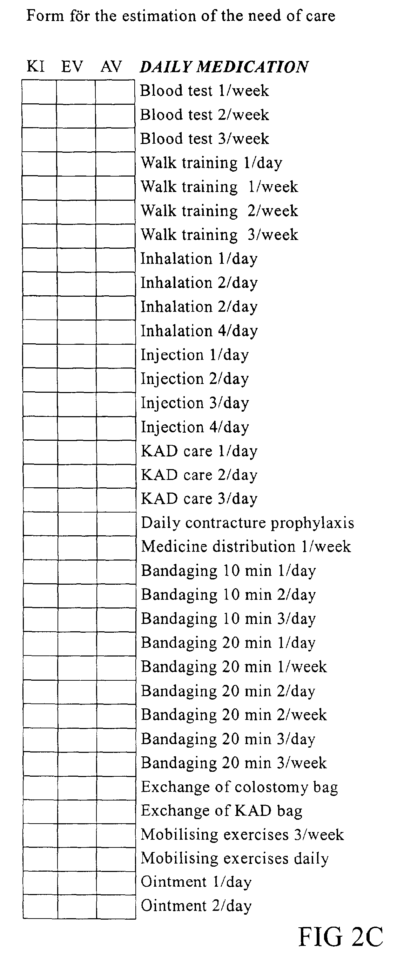

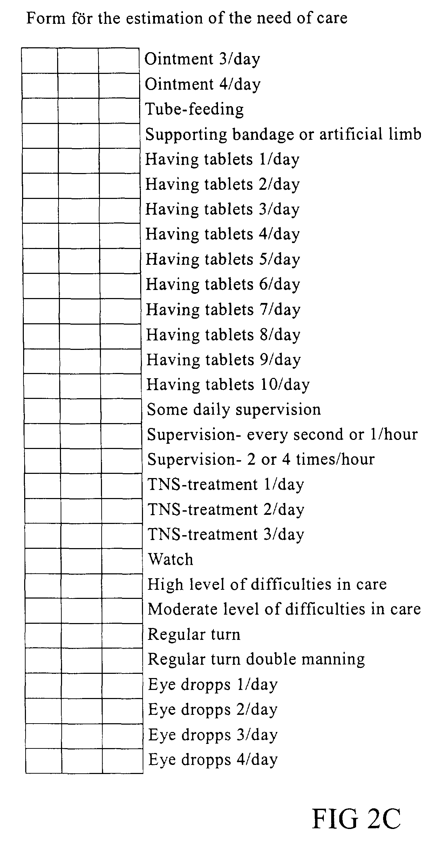

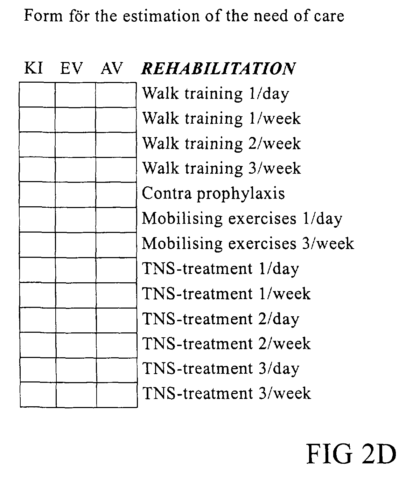

Different indices (points) have been determined from the level of needs for the activities in the care steps, and for the needs of medical and rehabilitation efforts. These are developed by time studies of the need of care. FIGs 2A and 2B illustrate examples of a form for points with respect to need of care and service, according to the steps 9-0 in the care steps. FIGs 2C and 2D illustrate examples of a form for points with respect to medical needs and needs of rehabilitation. The form is either a paper form, which is filled in manually for later registration (scanning) in the computer system, or an electronic form for registration of data directly into the computer. The same thing applies for each form in the system.

Under the heading of "ABILITY TO MOVE", it is found a number of different levels of ability to move, on which a person can be. Each level gives a predetermined point. If several alternatives are possible, the points are added for these. The method is repeated for "PERSONAL CARE", "DRESSING", "MEALS", "SUPERVISION", "DIFFICULTIES IN CARE", "DAILY SERVICE NEEDS", "WEEKLY SERVICE NEEDS", "SERVICE NEEDS EVERY SECOND WEEK", "MONTHLY SERVICE NEEDS", "DAILY MEDICATION" and "REHABILITATION". Summations of points are, of course, performed automatically in the computer system implementation.

The method has a structural adapted system of points which is based on about 80 critical indicators (care variables), which reflect different need of care, service, and nursing. Each indicator has a point, a so-called correlate. These are developed by time studies of needs ofcare. The method is flexible, and new indicators can be added as the needs are changing.

Except for the above-mentioned indicators, there is space for personal needs of transportation, administration and personal time - PA-indices. These are measured in a number of activity studies, and later on, an index is created for each care unit/activity/housing and the whole organization.

The method has a structural adapted system of points, which is based on about 80 indicators (need variables). These reflect different need of care, service and nursing. The need variables run in three equal sets of parallel tables abbreviated; KI, EV, AV (the so-called KIEVAV table), see FIGs 2A, 2B, 2C and 2D. The care and nursing efforts of the town districts and the private entrepreneur are registered in Table 1, abbreviated KI, the ability of the person receiving care to satisfy its needs is registered in Table 2, i.e. the level of functioning and self care, abbreviated EV, and the support and efforts of the relatives, abbreviated AV, are registered in Table 3. Then, from these tables and other tables with information stored in a data base, it can be gathered how the health development of the elder population with respect to age and sex and the support efforts of the relatives are changed over time.

Each indicator has a point as mentioned above, a so-called correlate which reflects different need of care, service, and nursing with respect to the individual, on the structure, the current housing and activity. In order to develop and illustrate the invention, intermittent, afterwards by means of the new computer technology continuously - measurements/studies have been performed in a town district of trial having about 900 persons receiving care. The study has been going on for about 7 years. The developed method of measurement as described above was used in the study with all variables in the clock of needs. The measurements show for example the care load, level of need of the persons living in a nursing home and the work load/full-year worker, and the need of resources for the individual person receiving care and the nursing unit.

Corresponding measurement with respect to the relation between needs and resources connected to detail studies, and the ability of functioning is not shown before the so-called elder reform. These measurements are the very first measurements performed and constitute a basis for comparison for subsequent measurements of care load and studies of different functions.

Complementary indices regarding for example mental and cultural needs can also be incorporated in a method according to the invention.

About 90 (with the possibility of more as well as less) physical and social activities, medical and rehabilitation efforts and the function continence are a part of the present embodiment of the invention. These are bathing, dress and undress, toilet, movement, food intake, bed making, breakfast, snack, walk, accompany, cooking, cleaning, purchase, security alarm, bank errands, medical and rehabilitation efforts and effect the direct part of the care. Additionally, about 10 (with the possibility of more as well as less) personnel related activities, such as transportation, administration, education and guidance, union activities are included.A map-out of the different needs of each person receiving care and an estimate of the grade of dependence according to a determined scale, i.e. the care steps, are included in a measurement. At the same time, a measurement of the time spent is performed in relation to each need and at what time of the day and night the different needs are most frequent. The study has been performed in a town district with about 25 000 inhabitants. The object of the study is partly to create a standard for different needs and other variables, which are included in the direct and indirect care, and an index for the activities in the activities of the town districts present for elder, disabled and sick persons. This is achieved by a validation and rehabilitation test together with an associated time study, in which the different activities are weighted against each other. There is an index for physical care, nursing, and rehabilitation efforts, a so-called nursing index and an index for social service efforts, a so-called service index, as well. There is an index for each individual activity, an all-in-all index for the care and service, respectively, and an index for the total care as a whole, i.e. the care and the service.

The nursing index, service index and care index is obtained by the following expression:

(need of nursing)/(number of persons receiving nursing)) = level of need of nursing    1

(need of service)/(number of persons receiving service) = (level of need of service)    2

need of care/(number of persons receiving care) = (level of need of care)    3

The index is given in points, which can be read from each form recorded in the computer.

With this measurement as a basis, a fair distribution of available resources can be obtained. It is done in such a way that, irrespective where in the town district a person lives, the allocated resource will be equally large based on the need of care.

As mentioned before, the object of this measurement is to create an index for care and service for the town district as a whole and to obtain a bases, a so-called need index, for optimum resource distribution. A B index, which is expressed in work load on a group level, is obtained by the following expression:

(level of need of care)/(care resource/person receiving care) = (work load)/(full-year worker)    4

resource - (indirect care+transport charges) = care resource    5

wherein the resource, indirect care and transport charges in the above expression are recounted from minutes to the number of full-year workers.

On a town district level, the work load is expressed in a value, which is the total needs in relation to available resources. The N value gives the average work load for the whole town district and constitutes the work load which is allowed by the current budget of the town district. The N value becomes the objective of the future follow-up of needs and available resources. Additionally, the N value forms the zero point where there is a balance between needs and resources.

Indices exists for both care, nursing, rehabilitation and service as a whole, but also for each separate activity. If for example a situation appears with reduced resources, the reducxtion can be distributed equally. With a method according to the invention, it is also easy to see what the economical outcome will be and whether any activity has to be deleted in order to release necessary resources, if the needs and/or the number of persons receiving care increase.

The N value or average work load is calculated by the following expression:

$$\left(\sum_{n=1}^{number} B_n\right)/\text{number} = \text{average work load} \qquad ..6$$

wherein the number is the number of units for which a B index has been calculated, for example the number of care groups or blocks of service flats.

With a calculated average work load and a current level of need, (a recommended care resource/person receiving care can be calculated from the following expression:

(level of need of care)/(average work load) = (recommended care resource)/(person receiving care) 7

The above-mentioned calculation of nursing, service, care and B index and N values on different levels are calculated by the computer system based on information registered from the forms.

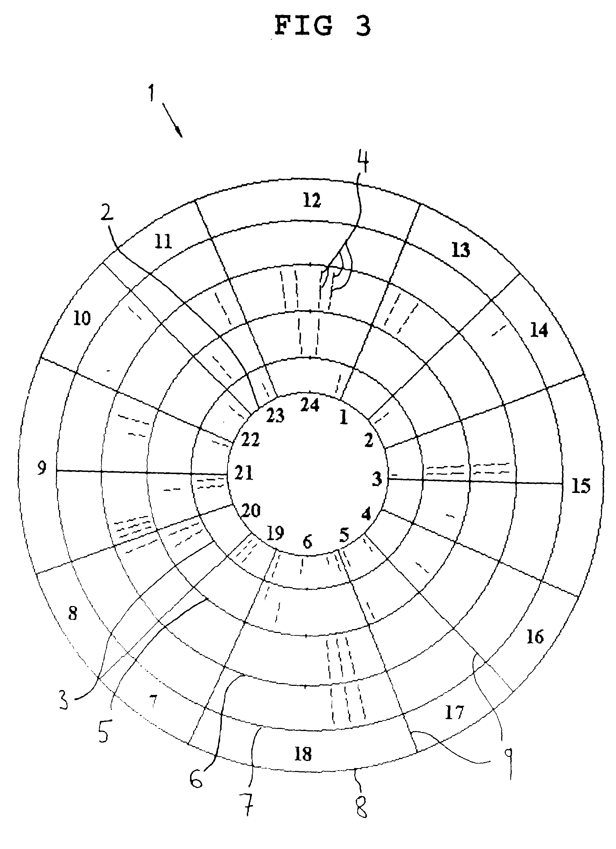

The needs of a person receiving care varies during day and night hours. In order to illustrate these needs in an easy and appropriate way, a clock of needs 1 in FIG 3 is used, which can be displayed graphically on a screen display connected to the computer system. In the inner first circle 2 of the clock 1, the 12 of the 24 hours of the day and night, which represent the evening and night, are marked by the numbers 19-24 and 1-6. Needs of a person receiving care during these hours are essentially physical needs. The physical needs, which usually are provided for during the evening and the night, are represented by the steps 9-6 in the first part of the care steps. The first circle 2 and a second circle 3 delimit the area, in which the physical needs are given. As mentioned above, each need is measured in points and each of these points is represented by a small mark 4 in the clock of needs.

A third circle 5 and the second circle 3 delimit an area including marks 4 representing points regarding physical needs (steps 9-4 in the care steps) during day time.

Nursing, such as medication and rehabilitation, which are performed by an assistant nurse, are represented by marks 4 in an area, which is delimited by a fourth circle 6 and the third circle 5.

Finally, points for the steps 3-0 in the care steps, i.e. the social needs, are represented by marks 4 in an area delimited by a fifth circle 7 and the fourth circle 6.

At which time during the day that the above-mentioned treatments have to be performed can be read from the 12 of the 24 hours representing the day and are marked by the numbers 7-18 in an area delimited by a sixth circle 8 and the fifth circle 7. Each hour of the clock 1 is delimited by lines 9, which extend from the outermost circle 8 to the innermost circle 2, whereby each hour forming a sector of a circle. Thus, the needs indicated by marks 4 in the areas delimited by the circles 5 and 3, 6 and 5, 7 and 6, respectively actions to be performed during the daytime, and needs, indicated by marks 4 in the area delimited by the circles 3 and 2 activities to be performed during the evening and the night.

Within the scope of the invention, it is possible to have other embodiments, wherein the grouping of the needs are more or less divided. In this embodiment, the second circle 3 together with the first circle 2 delimit an area, in which points for the needs (not only physical needs) during the evening and the night are given by marks 4. Further, the area delimited by the second circle 3 and the third circle 5 comprises marks 4, representing points regarding all needs during the daytime. In the same way as described earlier, the sixth circle 8 together with the fifth circle 7 delimits an area including the numbers 7-18, regarding the 12 of the 24 hours of the day and night representing the day.

In order to clearly mark what needs to be provided for during the evening and the night, the strokes of the clock, which are within the circle 2 in the embodiment described earlier, can be located in a new area, which is not shown on the drawings, outside the area delimited by the circles 3 and 2, in another embodiment.

In an alternative embodiment, the clock of needs 1 is completed with additional circles relating to for example mental and cultural needs.

For each level - from an individual person receiving care, group of persons receiving care, nursing home, group of nursing homes up to a whole town district - a clock of needs exists. The difference between the clocks of needs on different levels is that each mark 4 on the lowest level (an individual person receiving care) represents one point while each mark 4 on the next higher level represents for example 5 or 10 times more points than on the next lower level and so on. It is also possible to have a clock of needs, representing an even larger area, for example an administrative province or the total need of care in a whole country.

One or several computer systems for entering data can be present on each level and be connected by a network. This resulting in that the data are entered on the lowest level and are aggregated up to all represented levels.

The clock of needs can either be watched directly on the screen display of the computer system or be printed out on a paper. The result displayed by the clock of need is then used on different levels in order to distribute resources and satisfy the needs of an individual person receiving care in each situation.

By means of the clock of needs and the method according the invention, it is possible to compare the needs between for example two different nursing homes in order to distribute the resources. It is also possible to compare the needs of one nursing home with the needs of the administrative province or the country as a whole.

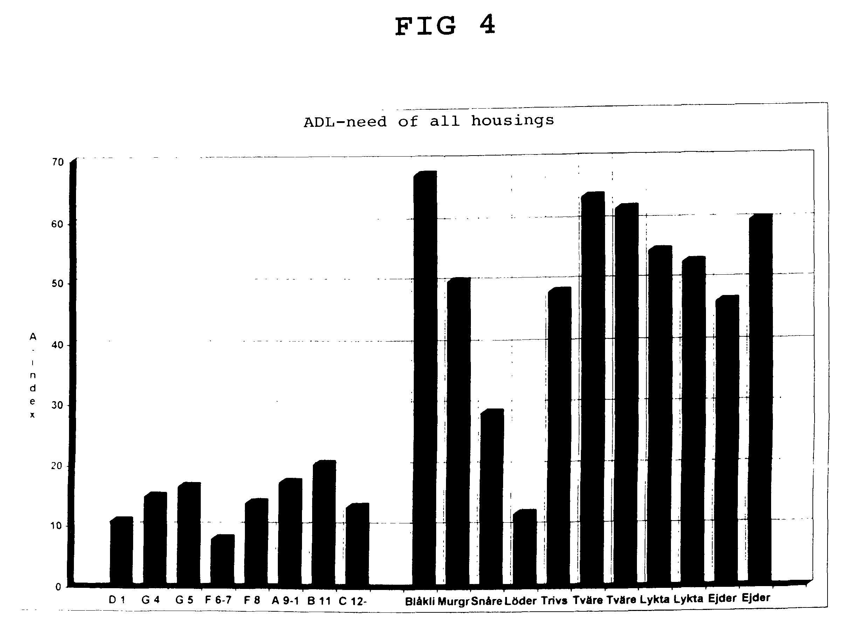

FIGs 4-7 illustrate examples of results, provided with the method according to the invention, based on measurements performed in a town district.

A bar graph is shown in FIG 4, where each of the first 8 bars from the left in the figure represents a home care group and the following 11 bars represents blocks of service flats. The height of the bars represents the care index (points) and shows the average need of care in all home care groups and blocks of service flats. The bars in FIG 4, which are marked by the same names, refer to different departments within the same block of service flat.

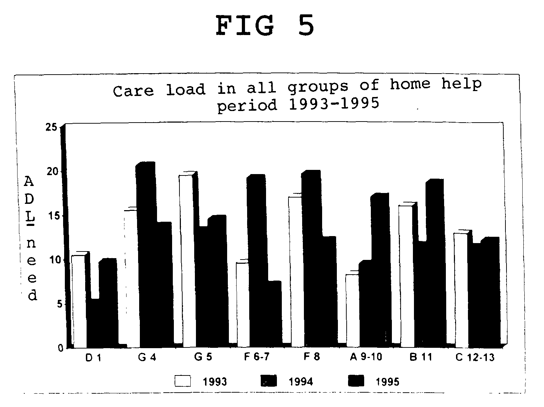

The care load (physical care needs) in all home-care groups in a town districts for a period of three years is shown in FIG 5. It is apparent from the diagram that the care load can vary very much both within and between different groups.

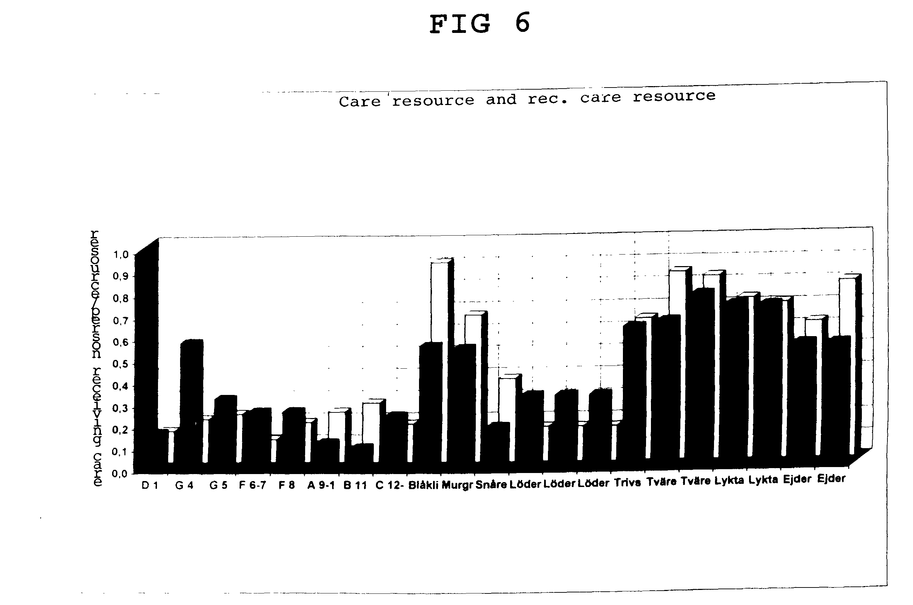

In the same way as in FIG 4, home care groups and blocks of service flats, respectively, are represented by bars in FIG 6. The height of the dark bars in the front corresponds to care resources/person receiving care, which is obtained by the expression 3 above.

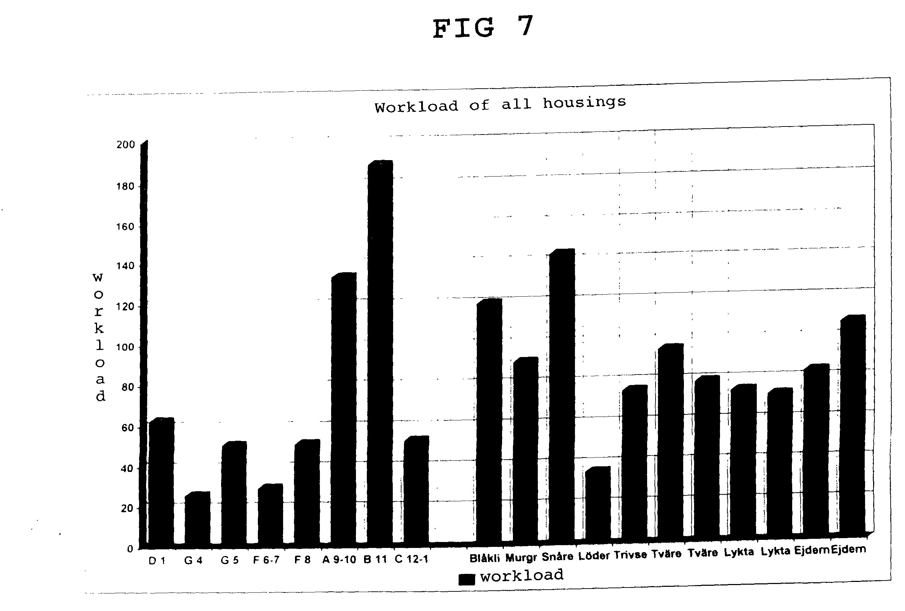

In FIG 7, it is shown a bar graph wherein the height of the bars corresponds to the work load/full-year worker for the same home care group and block of service flats as in the earlier graph. By using the values for the work load/full-year worker and calculate an average work load, which together with the level of need of the whole town district is inserted in the expression 7 above, a recommended care resource/person receiving care is obtained. A recommended care resource/person receiving care based on the values in FIG 4 and 7 of the white bars is illustrated in FIG 6. Further, it appears from FIG 6, that six home care groups and one block of service flats (Löderup) have more resources than allowed by the B index. Additionally, the home care groups and other housings need to be provided with resources.

Thereby the method and arrangement according to the invention solve one of the most important problems in the care and nursing, namely that in an objective way reflect the real conditions for the personnel and persons receiving care, i.e. to be able to measure the load of the individual needs of activities in relation to applied resources and then redistribute resources. This also results in that a balance is created, uneven distribution of resources is prevented, and it becomes easy to overview the activity. The present invention also gives the possibility of following up and evaluate the medical and economical development on different levels within the own organization - from the field activity and the individual person receiving care to meet the political decisions and the commission from the tax payers. As mentioned above, it will also be possible to perform comparing studies within and between town districts in different aspects.

By that, there are methods, which have been missed, for reflecting the real conditions for personnel and persons receiving care in the elder care and care of the disabled objectively.

From the point of view of the person receiving care, the balance between the need of resources and the resource take-out is important in order to guarantee a good qualitative care even in times of shrinking economic resources. It is an important aspect of working environment for the personnel to have a reasonably equally distributed work load irrespective of where in the organization you have the place of work.

The system according to the invention can be used in nursing institutions as well as in home help service and in home nursing for evaluation and introduction of for example assistance and subscription for home nursing in relation to the need of care of the person receiving care. The improved method according to the invention can also be used for evaluating the right to and the size of subscription for the relatives.

## Claims

1. A method of measuring the need of care and service in elder care and care of the disabled for efficient distribution of resources for need of care and service in for example elder care and care for the disabled, **characterized by** the steps of:

registering available resources as full-year workers and current need of each person receiving care;
determining a care and service index for each person receiving care and an evaluation of the level of dependence according to a predetermined scale;
determining a PA index (personnel and administration), relating to a cost counted in the number of full-year workers for indirect nursing, and a transportation time for each care unit;
determining a B index, relating to the work load per full-year worker in relation to provided resources of a care unit;
calculating an average work load in an N value, which is the total needs in relation to the available resources of the organization;
calculating a recommended care resource/person receiving care; and
applying a recommended care resource/person receiving care.

**2.** A method according to claim 1, wherein the care index is calculated according to the following expression:

need of care/(the number of persons receiving care) = the level of the need of care.

**3.** A method according to claim 1 or 2, wherein the B index is calculated according to the following expression:

the level of the need of care/(care resources/person receiving care) = the work load/full-year worker

wherein the resource-(indirect care+transport charges) = care resources.

**4.** Method according to any of the preceding claims, wherein the N value is calculated according to the following expression:

$$\left(\sum_{n=1}^{\text{number}} B_n\right)/\text{number} = \text{the average work load}$$

where the number is the number of units for which a B index is calculated.

**5.** A method according to any of the preceding claims, wherein the care resource/person receiving care is calculated according to the following expression:

level of need of care/average work load = recommended care resource/person receiving care.

**6.** A method according to any of the preceding claims 1-5, wherein the resource, the indirect cost for care and transportation costs are presented in the number of full-year workers.

**7.** A system for efficient distribution of resources for need of care and service in for example elder care and care of the disabled, comprising a computer system with memory means for storing program instructions and data, and a control unit operatively connected to the memory means, **characterized in that** the computer system further comprises:

means for registering of available resources as full-year workers as well as the current need of each person receiving care;
means for determining a nursing and service index for registered needs for each person receiving nursing and service and an evaluation of the level of dependence according to a predetermined scale;
means for determining a total need index, i.e. care, nursing, rehabilitation, and service needs, a so-called care index for each person receiving care according to an evaluation of a predetermined scale;
means for determining a PA index, an index for each care unit, which relates to a cost counted in the number of full-year workers for indirect care and transportation time,
means for determining a B index regarding work load/full-year worker, which is the total need in relation to applied resources:

means for calculating an average work load in an N-value, which is the total need in relation to available resources; and
means for calculating a recommended care resource/person receiving care.

**8.** A system according to claim 7, wherein the care index is calculated according to the following expression:

need of care/(number of persons receiving care) = level of need for care.

**9.** A system according to claim 7 or 8, wherein the B index is calculated according to the following expression:

the level of need of care/(care resources/person receiving care) = the work load/full-year worker

wherein the resource-(indirect care + transport charges) = care resources.

**10.** A system according to any of the claims 7-9, wherein the N value is calculated according to the following expression:

$$\left(\sum_{n=1}^{\text{number}} B_n\right)/\text{number} = \text{the average work load}$$

where the number is the number of units for which a B index is calculated.

**11.** A system according to any of claims 7-10, wherein the care resource/person receiving care is calculated according to the following expression:

level of need of care/average work load = recommended care resource/person receiving care.

**12.** A system according to any of the claims 7-11, wherein the calculated need is represented in a clock of needs (1) comprising:

a first circle (2) within which the 12 of the 24 hours of the day and night representing the evening and night are indicated by the numbers 19-24 and 1-6;
a second circle (3), which together with the first circle (2) delimits an area, in which points for needs during the evening and the night are indicated as marks (4) ;
a third circle (5), which together with the second circle (3) delimits an area including marks (4), representing points with respect to needs during the daytime; and
the sixth circle (8), which together with the fifth circle (7) delimits an area comprising numbers 7-18, relating to the 12 of the 24 hours of the day and night representing the day.

**13.** A system according to any of the claims 7-11, wherein the calculated needs are shown in a clock of needs (1), comprising

a first circle (2), within which the 12 of the 24 hours of the day and night representing the evening and night are indicated with numbers 19-24 and 1-6;
a second circle (3), which together with the first circle (2) delimits an area, in which points for physical needs are indicated by marks (4) ;
a third circle (5), which together with the second circle (3) delimits an area including marks (4) representing points, relating to physical needs during the daytime;
a fourth circle (6), which together with the third circle (5) delimits an area including marks (4) representing points, relating to nursing efforts;
a fifth circle (7), which together with the fourth circle (6) delimits an area including marks (4) representing points, relating to social needs; and
a sixth circle (8), which together with the fifth circle (7) delimits an area including numbers 7-18, relating to 12 of the 24 hours of the day and night representing the day.

**14.** A system according to claim 14, wherein the need clock (1) comprises two additional circles, which delimit an area including marks representing points, relating to mental and cultural needs, respectively.

**15.** A data program product, comprising a computer readable medium stored with a computer program code, **characterized by** making a computer perform the steps in any of the claims 1-6.

**16.** A computer program element, comprising a computer program code, **characterized by** making the computer execute the steps in any of the claims 1-6.

4 bath/shower

5 dressing

6 toilet

7 movment

8 continence

9 food intake

FIG 1A

0 cleaning

1 buy food

2 transport

3 cooking

FIG 1B

Form för the estimation of the need of care

*Department or care unit:*____________________ *Ordinary housing*

*Name:*__________________ *Personal code number:* __:__:__:__

| KI | EV | AV | *ABILITY TO MOVE* |
|---|---|---|---|
| | | | Only walks under supervision. |
| | | | Walks, does not need to be lifted |
| | | | Walks with natural support |
| | | | Wheel chair- help to drive outdoors |
| | | | Wheel chair- help to drive indoors |
| | | | Wheel chair- help in and out |
| | | | Bedridden |

| KI | EV | AV | *ABILITY TO MOVE* |
|---|---|---|---|
| | | | Faecal incontinence- several times a day |
| | | | Faecal incontinence- a few times a day |
| | | | Bath/shower |
| | | | Bedpan or bottle in the daytime |
| | | | Shaving |
| | | | Teeth or denture cleaning |
| | | | Washing - help with everything |
| | | | Washing - help with certain details |
| | | | Urinary incontinence several times a day (without KAD) |
| | | | Urinary incontinence a few times a day |
| | | | Go to the lavatory, help with movement |
| | | | Go to the lavatory, help with dressing |
| | | | Go to the lavatory, help with the cleaing |

| KI | EV | AV | *DRESSING* |
|---|---|---|---|
| | | | Dressing - help with all dressing, double manning |
| | | | Dressing - help with all dressing |
| | | | Dressing - help with some undressing |
| | | | Dressing - help with some dressing |

FIG 2A

Form för the estimation of the need of care

| KI | EV | AV | ***MEALS*** |
|---|---|---|---|
| | | | Feed - normal time spent |
| | | | Feed- exceptional time-consuming |

| KI | EV | AV | ***SUPERVISION*** |
|---|---|---|---|
| | | | Supervision- can be left for about an hour |
| | | | Supervision- daily |

| KI | EV | AV | ***DIFFICULITIES IN CARE*** |
|---|---|---|---|
| | | | Difficulties in care - high level |
| | | | Difficulties in care - moderate level |

FIG 2A

Form för the estimation of the need of care

| KI | EV | AV | *SERVICE NEEDS DAILY* |
|---|---|---|---|
| | | | Bed-making |
| | | | Breakfast |
| | | | Walk with the dog |
| | | | Afternoon coffee |
| | | | Evening coffee |
| | | | Supper |
| | | | Accompany 10 min |
| | | | Accompany 20 min |
| | | | Accompany 30 min |
| | | | Accompany 40 min |
| | | | Accompany 50 min |
| | | | Accompany 60 min |
| | | | Distribution of food |
| | | | Snack 1/day |
| | | | Snack 2/day |
| | | | Snack 3/day |
| | | | Snack 4/day |
| | | | Accompany 10 min |
| | | | Prepare dinner |
| | | | Help from a personal assistant |
| | | | Walk for 35 min |
| | | | Walk for 40 min |
| | | | Walk for 60 min |
| | | | Security alarm |

FIG 2B

Form för the estimation of the need of care

| KI | EV | AV | *SERVICE NEEDS EVERY WEEK* |
|---|---|---|---|
| | | | Bank errands |
| | | | Animal care |
| | | | Care of the cloths |
| | | | Distribution of food |
| | | | Buy food |
| | | | Menu planning - order, dinner |
| | | | Walk for 30 min once |
| | | | Walk for 30 min twice |
| | | | Walk for 30 min 3 times |
| | | | Walk for 45 min once |
| | | | Walk for 45 min twice |
| | | | Walk for 45 min 3 times |
| | | | Walk for 60 min once |
| | | | Walk for 60 min twice |
| | | | Walk for 60 min 3 times |
| | | | Cleaning |
| | | | Cleaning 80 min |
| | | | Cleaning contract |

| KI | EV | AV | *SERVICE NEEDS EVERY SECOND WEEK* |
|---|---|---|---|
| | | | Bath/shower |
| | | | Bank errands |
| | | | Care of the cloths |
| | | | Buy food |
| | | | Walk for 30 min |
| | | | Walk for 40 min |
| | | | Walk for 60 min |

| KI | EV | AV | *SERVICE NEEDS EVERY MONTH* |
|---|---|---|---|
| | | | Bank errands |
| | | | Care of the cloths |
| | | | Cleaning |
| | | | Other business |

FIG 2B

Form för the estimation of the need of care

| KI | EV | AV | *DAILY MEDICATION* |
|---|---|---|---|
| | | | Blood test 1/week |
| | | | Blood test 2/week |
| | | | Blood test 3/week |
| | | | Walk training 1/day |
| | | | Walk training 1/week |
| | | | Walk training 2/week |
| | | | Walk training 3/week |
| | | | Inhalation 1/day |
| | | | Inhalation 2/day |
| | | | Inhalation 2/day |
| | | | Inhalation 4/day |
| | | | Injection 1/day |
| | | | Injection 2/day |
| | | | Injection 3/day |
| | | | Injection 4/day |
| | | | KAD care 1/day |
| | | | KAD care 2/day |
| | | | KAD care 3/day |
| | | | Daily contracture prophylaxis |
| | | | Medicine distribution 1/week |
| | | | Bandaging 10 min 1/day |
| | | | Bandaging 10 min 2/day |
| | | | Bandaging 10 min 3/day |
| | | | Bandaging 20 min 1/day |
| | | | Bandaging 20 min 1/week |
| | | | Bandaging 20 min 2/day |
| | | | Bandaging 20 min 2/week |
| | | | Bandaging 20 min 3/day |
| | | | Bandaging 20 min 3/week |
| | | | Exchange of colostomy bag |
| | | | Exchange of KAD bag |
| | | | Mobilising exercises 3/week |
| | | | Mobilising exercises daily |
| | | | Ointment 1/day |
| | | | Ointment 2/day |

FIG 2C

Form för the estimation of the need of care

| | | | |
|---|---|---|---|
| | | | Ointment 3/day |
| | | | Ointment 4/day |
| | | | Tube-feeding |
| | | | Supporting bandage or artificial limb |
| | | | Having tablets 1/day |
| | | | Having tablets 2/day |
| | | | Having tablets 3/day |
| | | | Having tablets 4/day |
| | | | Having tablets 5/day |
| | | | Having tablets 6/day |
| | | | Having tablets 7/day |
| | | | Having tablets 8/day |
| | | | Having tablets 9/day |
| | | | Having tablets 10/day |
| | | | Some daily supervision |
| | | | Supervision- every second or 1/hour |
| | | | Supervision- 2 or 4 times/hour |
| | | | TNS-treatment 1/day |
| | | | TNS-treatment 2/day |
| | | | TNS-treatment 3/day |
| | | | Watch |
| | | | High level of difficulties in care |
| | | | Moderate level of difficulties in care |
| | | | Regular turn |
| | | | Regular turn double manning |
| | | | Eye dropps 1/day |
| | | | Eye dropps 2/day |
| | | | Eye dropps 3/day |
| | | | Eye dropps 4/day |

FIG 2C

Form för the estimation of the need of care

| KI | EV | AV | ***REHABILITATION*** |
|---|---|---|---|
| | | | Walk training 1/day |
| | | | Walk training 1/week |
| | | | Walk training 2/week |
| | | | Walk training 3/week |
| | | | Contra prophylaxis |
| | | | Mobilising exercises 1/day |
| | | | Mobilising exercises 3/week |
| | | | TNS-treatment 1/day |
| | | | TNS-treatment 1/week |
| | | | TNS-treatment 2/day |
| | | | TNS-treatment 2/week |
| | | | TNS-treatment 3/day |
| | | | TNS-treatment 3/week |

FIG 2D

FIG 3

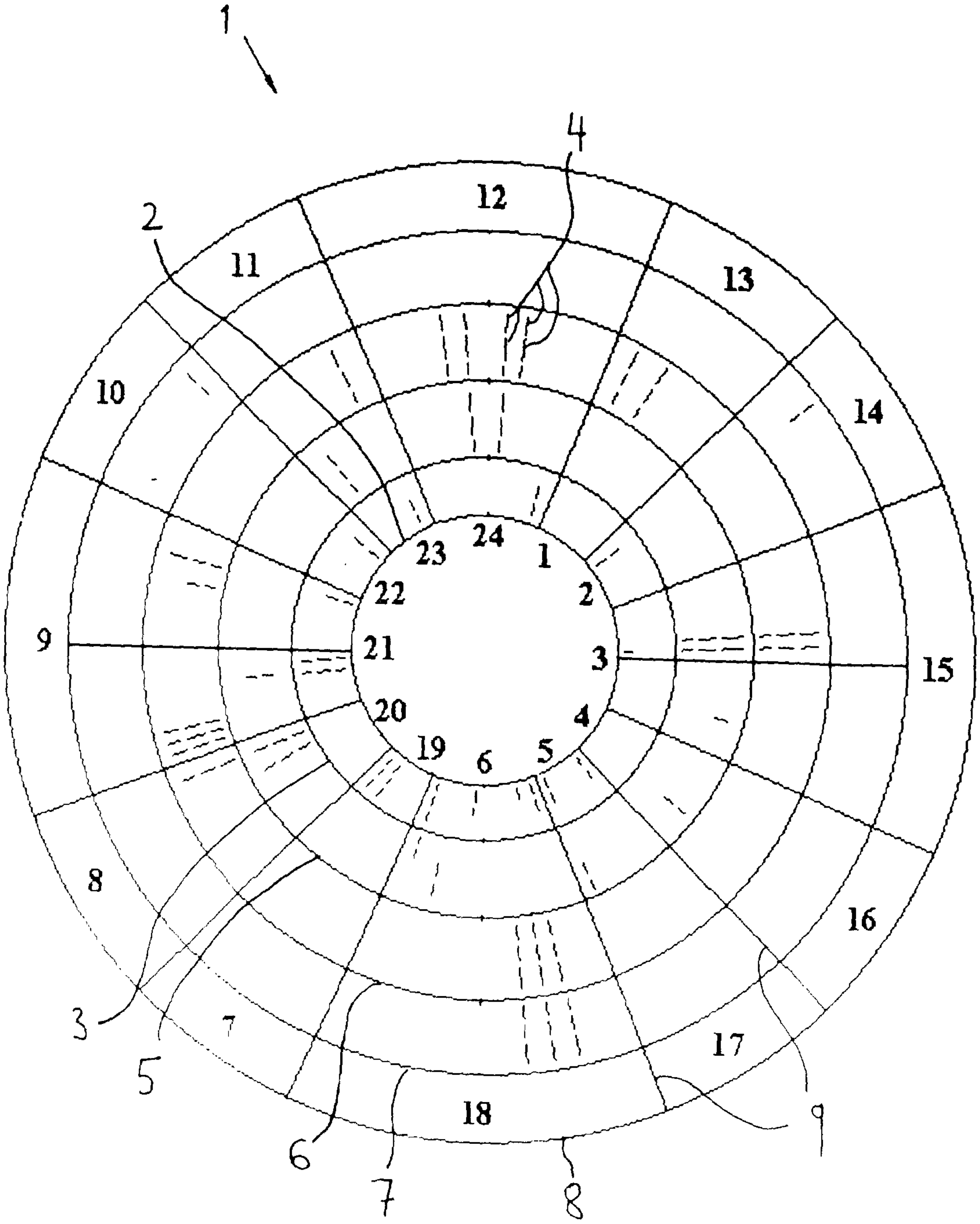

FIG 4

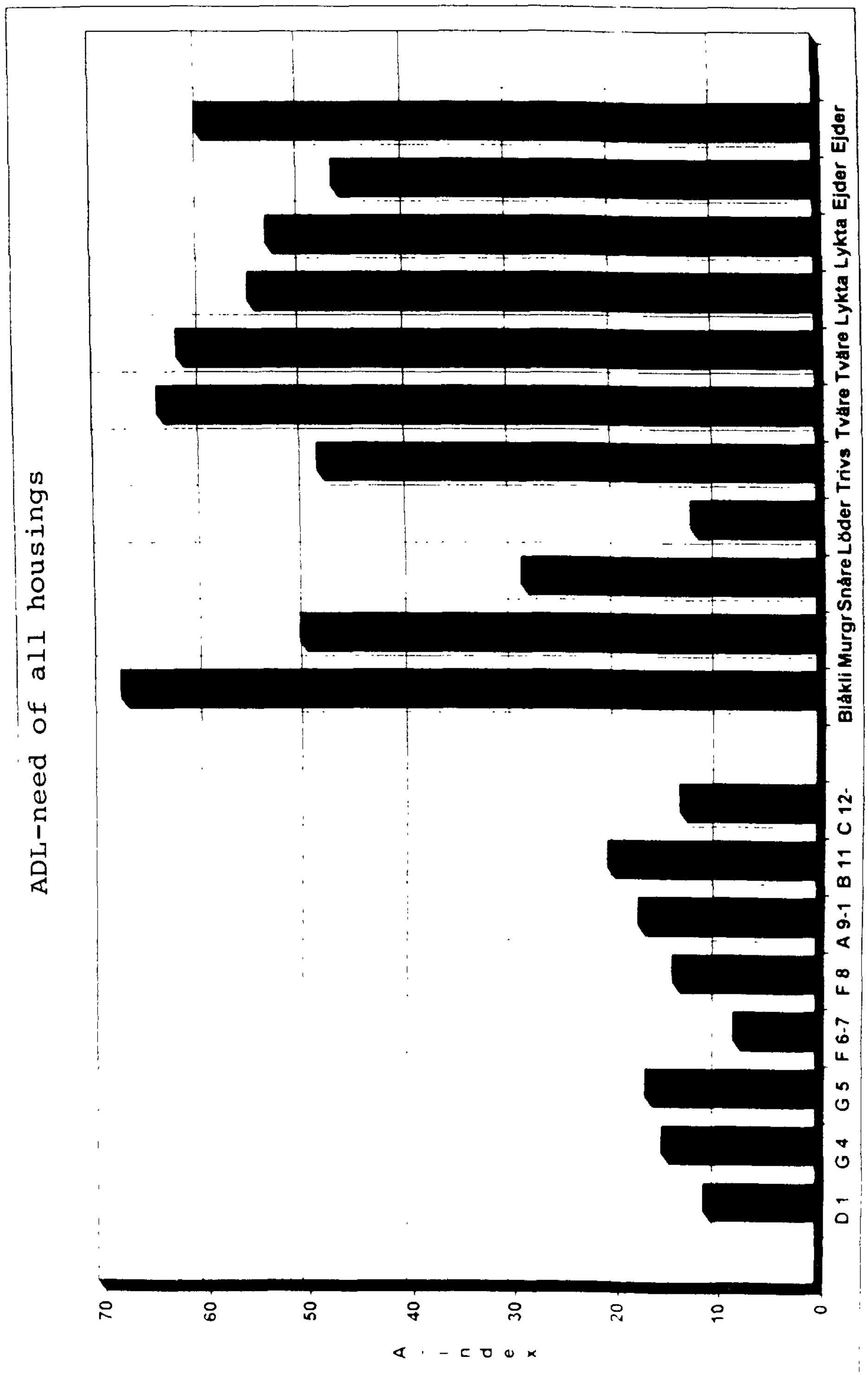
ADL-need of all housings
A-index
0
10
20
30
40
50
60
70
D 1
G 4
G 5
F 6-7
F 8
A 9-1
B 11
C 12-
Blåkli
Murgr
Snåre
Löder
Trivs
Tväre
Tväre
Lykta
Lykta
Lykta
Ejder
Ejder

FIG 5

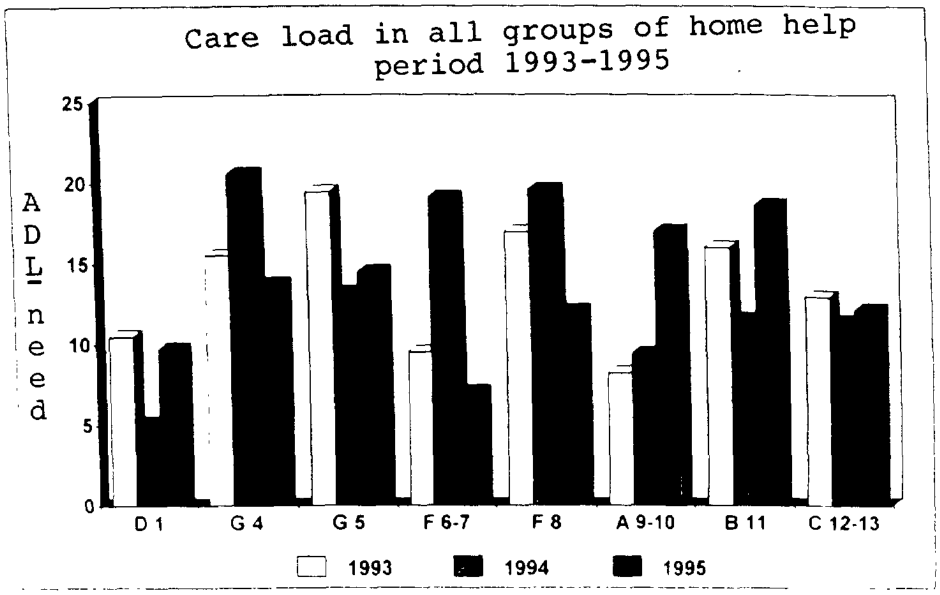
Care load in all groups of home help
period 1993-1995
ADL need
25
20
15
10
5
0
D 1
G 4
G 5
F 6-7
F 8
A 9-10
B 11
C 12-13
1993
1994
1995

FIG 6

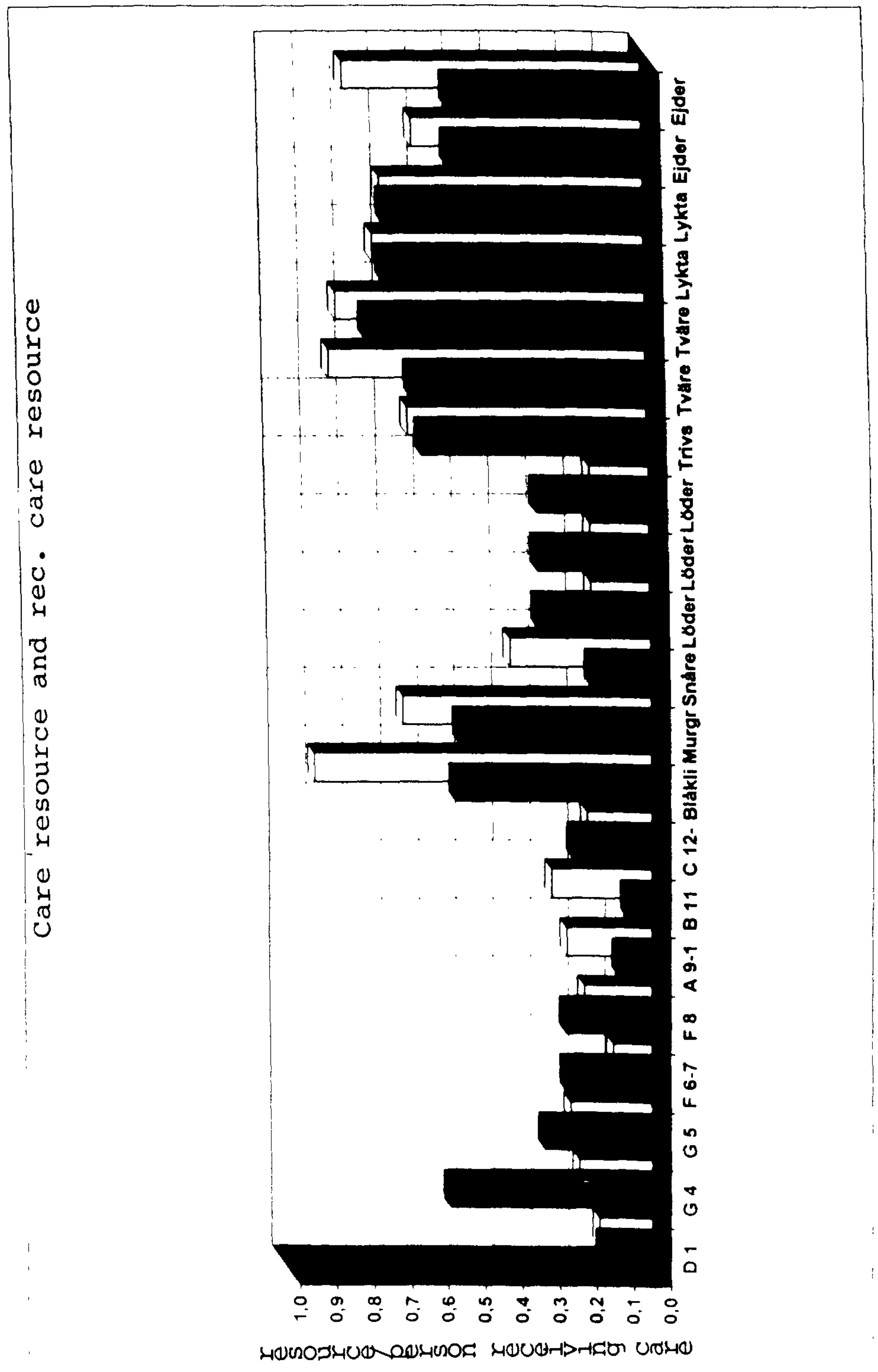
Care resource and rec. care resource
1,0
0,9
0,8
0,7
0,6
0,5
0,4
0,3
0,2
0,1
0,0
Resource/person receiving care
D 1
G 4
G 5
F 6-7
F 8
A 9-1
B 11
C 12-
Blåkli
Murgr
Snåre
Löder
Löder
Löder
Löder
Trivs
Tväre
Tväre
Lykta
Lykta
Ejder
Ejder

FIG 7

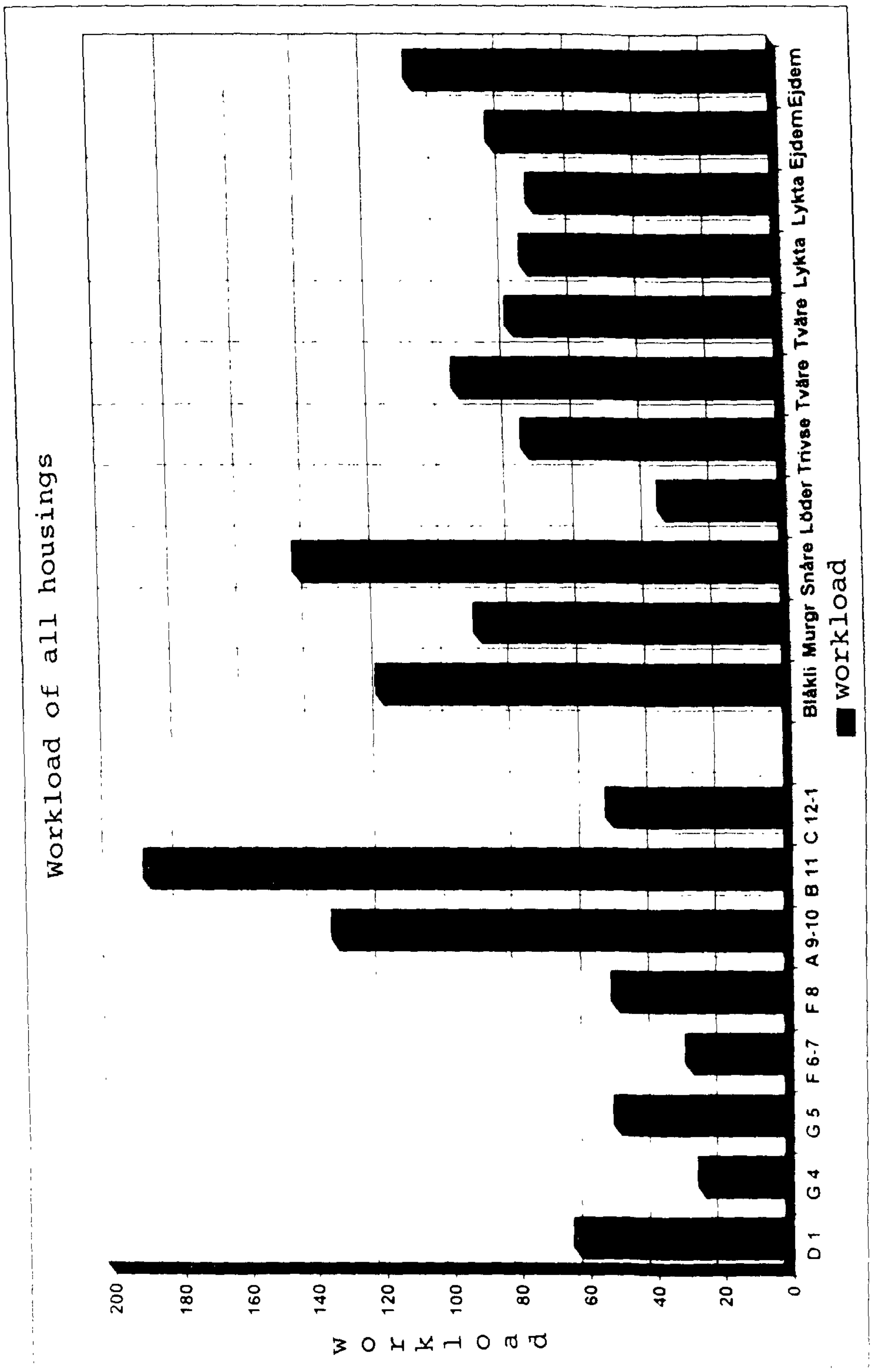
Workload of all housings
workload
200
180
160
140
120
100
80
60
40
20
0
D 1
G 4
G 5
F 6-7
F 8
A 9-10
B 11
C 12-1
Blåkli
Murgr
Snåre
Löder
Trivse
Tvåre
Tvåre
Lykta
Lykta
Ejdem
Ejdem
workload

European Patent Office

# EUROPEAN SEARCH REPORT

Application Number
EP 00 20 3290

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X<br><br>A | WO 97 40463 A (DEROYAL IND INC)<br>30 October 1997 (1997-10-30)<br>* page 3, line 25 - page 7, line 8 *<br>* page 12, line 20 - page 14, line 11; figure 3 * | 1,7,15,16<br>2-6,8-14 | G06F19/00<br>G06F17/60 |
| A | US 5 842 173 A (STRUM DAVID P ET AL)<br>24 November 1998 (1998-11-24)<br>* column 3, line 9 - column 4, line 67 *<br>* column 5, line 59 - column 17, line 6; figures 1-15 * | 1-16 | |
| A | WO 97 25682 A (HIRSCH JOHN D ;RODIN ERVIN Y (US)) 17 July 1997 (1997-07-17)<br>* page 4, line 7 - page 6, line 24 *<br>* page 10, line 1 - page 20, line 10; figures 1-14 * | 1,7 | |
| A | US 5 065 315 A (GARCIA ANGELA M)<br>12 November 1991 (1991-11-12)<br>* column 1, line 6 - column 10, line 19 * | 1,7 | TECHNICAL FIELDS SEARCHED (Int.Cl.7) |
| A | US 5 913 201 A (KOCUR GEORGE A)<br>15 June 1999 (1999-06-15)<br>* column 1, line 35 - column 4, line 3 * | 1,7 | G06F |

The present search report has been drawn up for all claims

| Place of search | Date of completion of the search | Examiner |
|---|---|---|
| THE HAGUE | 19 February 2001 | Schenkels, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 00 20 3290

This annex lists the patent family members relating to the patent documents cited in the above-mentioned European search report.
The members are as contained in the European Patent Office EDP file on
The European Patent Office is in no way liable for these particulars which are merely given for the purpose of information.

19-02-2001

| Patent document cited in search report | | Publication date | Patent family member(s) | | | Publication date |
|---|---|---|---|---|---|---|
| WO 9740463 | A | 30-10-1997 | CA | 2252698 | A | 30-10-1997 |
| | | | EP | 0981794 | A | 01-03-2000 |
| | | | JP | 2001500290 | T | 09-01-2001 |
| US 5842173 | A | 24-11-1998 | NONE | | | |
| WO 9725682 | A | 17-07-1997 | AU | 1693897 | A | 01-08-1997 |
| | | | EP | 0954812 | A | 10-11-1999 |
| US 5065315 | A | 12-11-1991 | AU | 6622090 | A | 31-05-1991 |
| | | | CA | 2067747 | A | 25-04-1991 |
| | | | EP | 0497832 | A | 12-08-1992 |
| | | | WO | 9106917 | A | 16-05-1991 |
| US 5913201 | A | 15-06-1999 | NONE | | | |

EPO FORM P0459

For more details about this annex : see Official Journal of the European Patent Office, No. 12/82